# EUROPEAN PATENT SPECIFICATION

(11) **EP 2 976 020 B1**
(45) Date of publication and mention of the grant of the patent: **21.08.2019**
(21) Application number: 14715697.0
(22) Date of filing: 18.03.2014
(51) Int. Cl.: A61B 34/30

(54) **A MINIATURE ROBOTIC DEVICE APPLICABLE TO A FLEXIBLE ENDOSCOPE FOR THE SURGICAL DISSECTION OF GASTRO-INTESTINAL TRACT SURFACE NEOPLASMS**
MINIATURISIERTE ROBOTISCHE VORRICHTUNG FÜR EIN FLEXIBLES ENDOSKOP ZUR CHIRURGISCHEN SEZIERUNG VON SUPERFIZIELLEN NEOPLASMEN DES MAGEN-DARM-TRAKTES
DISPOSITIF ROBOTIQUE MINIATURE POUVANT ÊTRE APPLIQUÉ À UN ENDOSCOPE SOUPLE POUR LA DISSECTION CHIRURGICALE DE NÉOPLASMES DE SURFACE DE TRACTUS GASTRO-INTESTINAL

(30) Priority: 18.03.2013 IT FI20130055
(43) Date of publication of application: 27.01.2016
(73) Proprietor: Scuola Superiore di Studi Universitari e di Perfezionamento Sant'Anna, 56127 Pisa (IT); Università Degli Studi Di Torino, 10124 Torino (IT)
(72) Inventor: AREZZO, Alberto, 10133 Torino (IT); RANZANI, Tommaso, 56121 Pisa (IT); MENCIASSI, Arianna, 56020 Pontedera (Pisa) (IT); BONINO, Marco Augusto, 10025 PinoTorinese (Torino) (IT); MORINO, Mario, 10133 Torino (IT); DARIO, Paolo, 56043 Lorenzana (Pisa) (IT)
(74) Representative: Brazzini, Silvia
(86) International application number: PCT/IB2014/059939
(87) International publication number: WO 2014/147556

(56) References cited:
- EP-A2- 1 946 707
- WO-A1-2007/111571
- WO-A1-2010/042611
- WO-A1-2010/138083
- US-A1- 2003 028 217
- US-A1- 2005 096 502

## Description

### Field of the invention

The invention, in general, refers to the field of mini-invasive surgery of the digestive tract and in particular it concerns the medical treatment of surface neoplasms of a tract of the digestive tract through endoluminal local excisions. More precisely, the invention concerns a miniature robotic device that is designed to be coupled to a flexible endoscope for the surgical dissection of gastrointestinal tract surface neoplasms.

### State of the art

Tumors of the digestive tract are one of the greatest challenges for national health systems worldwide, since they are a frequent cause of death and they lead to substantial economic burdens. Today, thanks to screening programs, these neoplasms can be diagnosed early. This makes it possible for there to be a less invasive medical treatment with respect to major surgery, which can be obtained through local excision with an endoluminal approach. This already occurs for lesions of the medium and upper rectum by means of a surgical method known as T.E.M. (Transanal Endoscopic Microsurgery), which uses the anal orifice as an access point and it is obtained through a rigid operating rectoscope with has had excellent results for already more than 30 years. However, due to its rigidity and bulk, this system requires the operation to be carried out in general anaesthesia and it cannot be used for operations in the lower proximal digestive tract, in particular in the colon.

For lesions of the colon, the endoscopic instruments available today do not generally allow surgical manoeuvring (manipulation, dissections, suture, etc.), since they were conceived for diagnostic purposes.

Recently numerous accessories have been made for the endoscopic resection aimed at overcoming the limitations of a purely diagnostic method, like flexible endoscopy was initially. Nevertheless, their use requires a learning curve that is long and complex; furthermore, the results of this method are not always brilliant.

One known flexible endoscopic method is represented by the Endoscopic Mucosal Resection (EMR), i.e. the removal through diathermy snare that is introduced through the operative channel of the flexible endoscope and upon lifting of the lesion through the infiltration of a saline solution in the submucosal layer of the wall. This method however, makes it possible for there to be an *en-bloc* excision only for lesions with dimensions that are up to 2 cm and it carries the burden of a high rate of local relapse.

An alternative known method is represented by the Endoscopic Submucosal Dissection (ESD), wherein such a method is again carried out by means of a flexible endoscope introducing special diathermic dissector scalpel through the operating channel. This method however, requires procedures that take several hours for lesions of even only 3-4 cm diameter, with a high risk of perforating the intestine (up to 18%) and a percentage of *en-bloc* excision that goes from 50 to 80% for lesions wider than 2 cm, according to the published experiences.

It is furthermore important to bear in mind that a critical point for a correct therapy is the diagnosis of the pathology on the resected tissue with the defining of the unharmed margins around the lesion. It has been observed that the lesions with a diameter greater than 20 mm, despite using the most recent methods, are totally resected in only around two-thirds of cases. For these reasons it is currently necessary to subject these lesions to a second invasive surgical treatment because there is not a safe oncological radicality.

Endoluminal surgery, using a flexible endoscope, is an additional medical option to the screening programs that make it possible to identify early neoplasms and precancerous lesions. Currently however, despite the use of up-to-date instruments that allow the endoscopic resection of the submucosa, a third of the early neoplasms or precancerous lesions with a diameter that is greater than 2 cm are removed in a way that does not satisfy the oncological requisites of medical resection.

Therefore, patients who undergo surgery treatment with an unsatisfactory outcome are necessarily sent on, at a later moment, to major abdominal surgery.

The endoscopic resection of lesions must be further actuated by highly competent and skilled surgeons since it is a procedure that is not without complications such as perforations and haemorrhages. Such complications are indeed possible especially due to the unsuitableness of the devices that are currently available with which endoluminal resections can be carried out.

In order to avoid such risks, many patients, who could benefit from an endoscopic treatment, are instead sent directly to have major resective surgery, with increased morbility, mortality and greater costs.

Surgical instruments are known, for example for laparoscopic surgery, having robotic arms, such as an ARTEMIS system (Schurr M.O., Arezzo A., Buess G.F., Robotics and systems technology for advanced endoscopic procedures: experiences in general surgery, Eur J Cardiothorac Surg. 1999 Nov; 16 Suppl 2:S97-105. Review), which consists of two robotic arms ending with two laparoscopic instruments that are controlled by a console that is provided with controls that are driven by the surgeon.

Endoluminal surgery devices that are equipped with robotic arms are also described in Menciassi A. et al., Miniaturized robotic devices for endoluminal diagnosis and surgery: a single-module and a multiple-module approach, Conf Proc IEEE Eng Med Biol Soc. 2009; 2009:6842-5.

The use of micro-robots introduced in the peritoneal cavity of pigs through a standard 12 mm laparoscopic trocar after CO₂ insufflation, was described in Oleynikov D. et al., Miniature robots can assist in laparoscopic cholecystectomy, Surg Endosc., vol. 19, pp. 473-476, DOI: 10.1007/s00464-004-8918-6, 2005.

WO 2007/149559 describes a surgical robotic device that can be inserted in the body of the patient and that can be steered within it by using magnets outside of the body. A pair of robotic arms protrudes from a body that contains magnets that interact with an external magnetic handle for controlling the position of the device. Every arm ends with a surgical instrument and is connected to the body through a shoulder joint with two degrees of freedom. Every arm further comprises two arm portions that are connected to one another through an elbow joint with one degree of freedom.

Another dissecting/manipulator robotic instrument comprising two robotic arms driven by small motors and an imaging source is described in US 2008/065105.

WO 2010/138083 describes a robotic manipulator for flexible endoscopy comprising a flexible element that is configured so as to be able to be coupled with an endoscope and at least one arm that is connected to one end of the flexible element, whereas another end thereof is connected through tie-rods to an actuation device for moving the arm. Since the end of the arm is completely exposed, in order to avoid damaging to the tissue and jamming of the equipment during the movement of the manipulator inside the body of the patient, a sort of sheath is used - a sheet - which covers the equipment, and can be removed once the instrument has reached the operative position.

WO 2007/111571 describes a robotic surgical manipulator for flexible endoscopy wherein a pair of robotic arms, ending with surgical instruments, extends from the distal end of a flexible endoscope. The insertion of this manipulator during an endoscopic surgical operation can take place by introducing the endoscope with the robotic arms already connected in the position of use, and exposed beyond the distal end of the endoscope. This makes it necessary, for safety reasons, to introduce the endoscope so equipped, inside of a tubular guide, or *overtube,* which is pre-positioned to the site of surgery; this procedure, besides being laborious, is feasible in the upper digestive tract (oesophagus and stomach), but only in part in the lower digestive tract (rectum and sigmoid).

US 2008/300460 describes a cylindrical cap that is suitable for being applied to the distal end of an endoscope and having a bellows-shaped portion that allows the cap to extend axially and to be oriented in directions other than the axial direction. The cap allows mucosa to be sucked so as to apply, for example, a high frequency loop that is inserted through the operative channel of the endoscope. WO 2010/041714 describes another cap that can be applied to the tip of an endoscope to perform ESD dissections.

### Summary of the invention

The general object of the present invention is to provide a miniature robotic device that is suitable for being integrated on a common commercial flexible endoscope that allows the surgical dissection of gastrointestinal tract surface neoplasms.

A particular object of the present invention is to provide a robotic device of the type mentioned above that comprises robotic arms for manipulating the tissue and its dissection so as to ensure that such a dissection is performed within margins free from disease.

Another object of the present invention is to provide a robotic device of the type mentioned above that makes it possible to prevent, as much as possible, and in any case to better manage possible bleeding or perforations.

A further object of the present invention is to provide a robotic device of the type mentioned above having small dimensions so as to minimise discomfort of the patient during surgery.

Yet a further object of the present invention is to provide a robotic device of the type mentioned above that is also suitable for use in transluminal surgery, or rather, that is suitable for always being introduced through natural orifices, therefore through the wall of the vagina, of the rectum or of the stomach, so as to access the peritoneal cavity.

Yet a further object of the present disclosure is to provide an exemplary method for the surgical treatment of the digestive tract by means of operations of endoluminal or transluminal surgery with a robotic device of the type mentioned above, which guarantees optimal safety both from the point of view of protection of bodily tissues where the device passes through both from the point of view of ease of insertion and use of the device itself.

These objects are achieved with the miniature robotic device that is designed to be coupled to a flexible endoscope according to the present invention the essential characteristics of which are outlined in claim 1. Further important characteristics are outlined in the dependent claims.

### Brief description of the drawings

The characteristics and the advantages of the robotic device according to the present invention shall become clear from the following description of an embodiment thereof given as an example and not for limiting purposes with reference to the attached drawings, in which:
- figure 1 is a perspective view of the robotic device according to the invention in its operative configuration inside the colon;
- figure 2 is an enlarged view of the operative end of the robotic device of figure 1;
- figure 3 is a longitudinal section view of the robotic device according to the invention;
- figure 4 is a front perspective view of the cap body containing the robotic arms;
- figure 5 is a perspective view of the robotic arms of the robotic device according to the invention;
- figures 6a and 6b schematically illustrate a possible way of actuating a robotic arm of the robotic device according to the invention;
- figure 7 illustrates a preferred actuation system for the sliding of the robotic arms of the device according to the invention;
- figures 8 and 9 schematically show longitudinal section views of possible embodiments of the manipulator arm and of the dissecting arm of the robotic device according to the invention.

### Detailed description of the invention

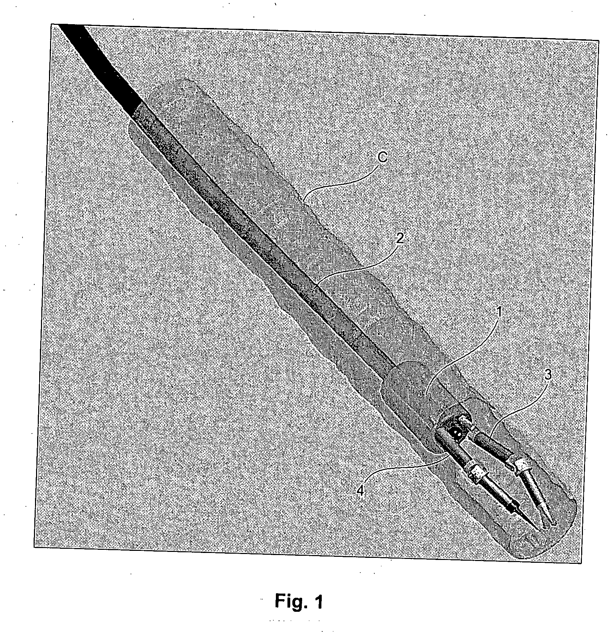

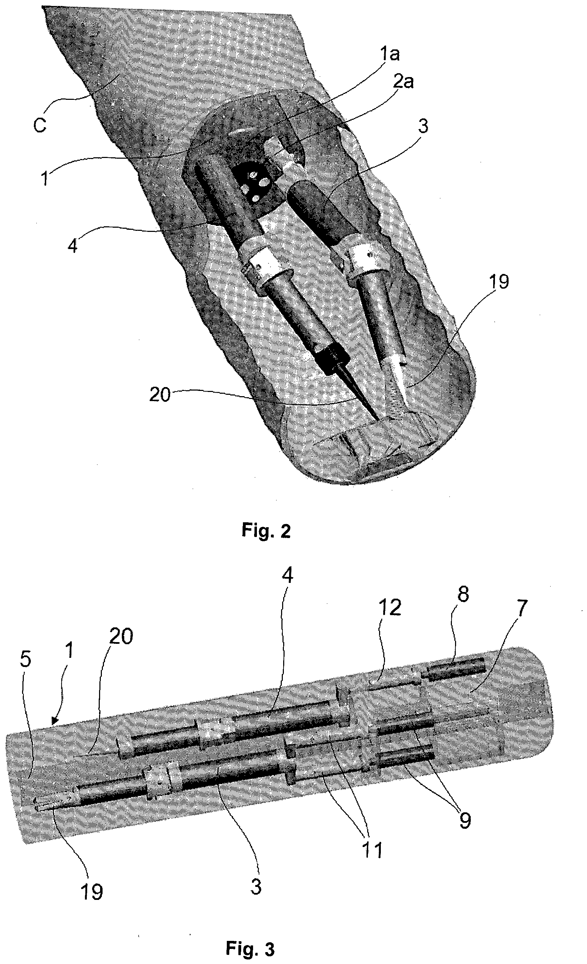

With reference to figures 1 to 4, the robotic device according to the invention schematically comprises a body 1 that is substantially shaped so as to be fixed like a cap to the distal end of a generic commercial endoscopic instrument 2 that is suitable for being used for explorations inside a tract of the gastrointestinal apparatus, for example of the colon, indicated with the letter C in the figures. The cap body 1 contains two robotic arms 3 and 4 that are respectively for manipulating (manipulator arm 3) and for the surgical dissection (dissecting arm 4) of superficial lesions of the upper digestive tract, lower digestive tract and for transluminal surgery. The two arms 3 and 4 are suitable for being arranged in a first position, or rest position, illustrated in figure 3, in which they are integrally contained retracted inside the cap body 1, and in a second position, or operative position, illustrated in figures 1, 2 and 7, wherein they protrude out from one end of the cap body 1. The arrangement of the arms in the first position makes it possible to easily reach the surgery site.

The cap body 1 has a substantially cylindrical shape that is suitable for being applied to the end of an endoscope that is commonly used in diagnostic practice (diameter of 5-10 mm). Inside this there is an axial channel 5 for the insertion of the distal end portion of the endoscope 2 and, around said channel 5, there is a chamber 6 for housing the arms 3 and 4 in the retracted position. Once said distal end portion of the endoscope 2 is housed in the cap body 1, its end, indicated with 2a in figure 2, with the outlet of its operative channels, the light source and the imaging system, faces the end of the cap body 1, said end also being called the front end and being indicated with reference numeral 1a.

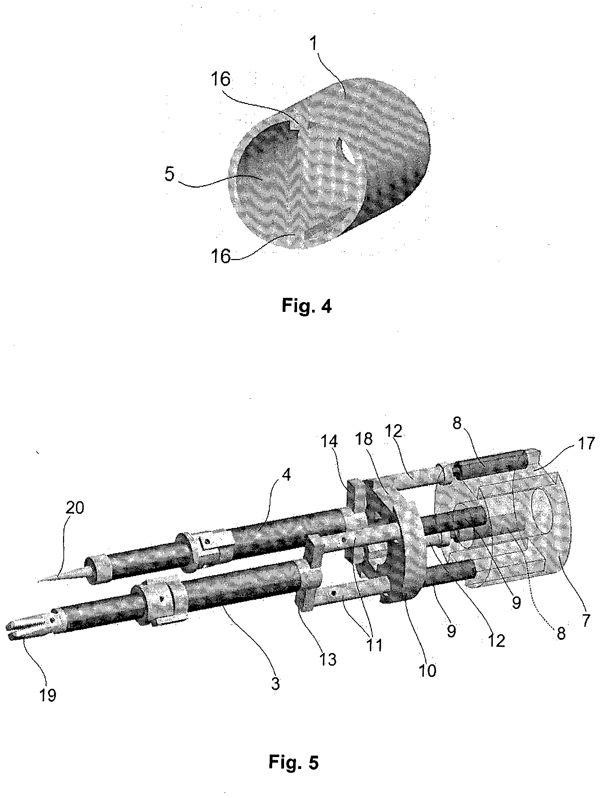

More precisely, with reference also to figure 5, in the chamber 6 of the cap body 1 an axially sliding base 7 is mounted, to which two linear actuator pairs 8 and 9 are fixed. The actuator pair 8 is associated with the dissecting arm 4 and is fixed inside the base 7, whereas the actuator pair 9, which are associated with the manipulator arm 3, is fixed on the base 7. A first pair of joint elements 11 protrudes from the actuator pair 8, whereas a second pair of joint elements 12 protrudes from the actuator pair 9. The first pair of joint elements 11 protrudes from a support plate 10 and connects to a crosspiece 13 forming the base of the manipulator arm 3. The second pair of joint elements 12 engages inside the support plate 10 and connects to a crosspiece 14 forming the base of the dissecting arm 4.

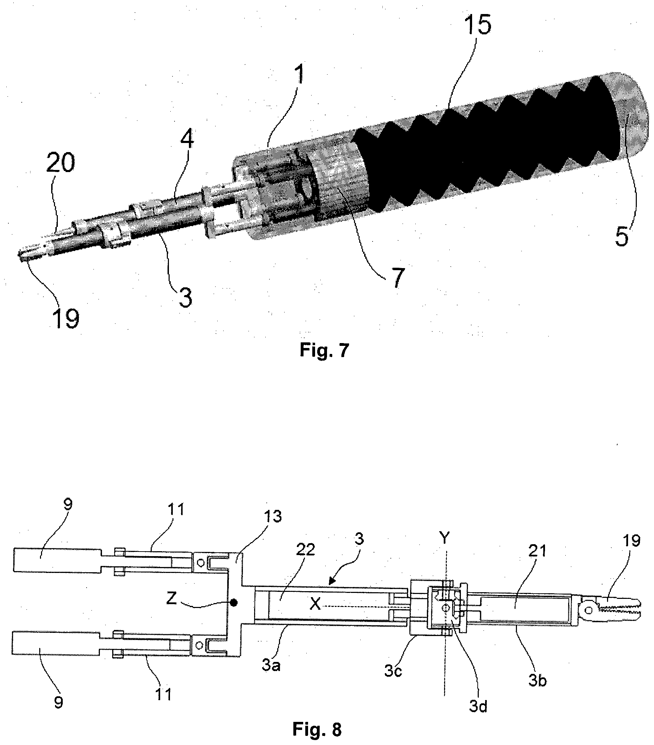

The axial sliding of the robotic device within the cap body 1, which allows the arms 3 and 4 to project out from the cap body 1, once the surgery site has been reached, is obtained through actuator means which, in the embodiment illustrated in figure 7, comprise a fluidic chamber 15 that is closed with a central channel that is coaxial with respect to the channel 5 of the cap body 1. The chamber 15 is arranged between the slidable base 7, to which it is fixed, and the proximal bottom end of the cap body 1 and it communicates with an external source, not shown, of fluid, gas or liquid. The chamber 15 is shaped (for example bellows-shaped) so as to expand or contract depending on whether it is filled or emptied of the fluid, whereby its expansion or contraction results in the sliding of the slidable base 7 and the consequent extension out or, respectively, retraction of the arms 3 and 4. In order to prevent the rotation of the device during the sliding, the latter is guided along the cap body 1 by means of longitudinal ribs 16 (figure 4) that engage with corresponding throats 17 on the base 7 of the device. Similar throats 18 with the same function are foreseen on the support plate 10.

The robotic arms 3 and 4 are articulated so as to provide the endoscope 2 with the capability of carrying out manipulations and dissections of surface neoplasms in the digestive tract. Their maximum external diameter, in a practical embodiment of the invention, is 6-8 mm and, once they are opened out, they protrude with respect to the end of the endoscope 2 by a maximum of 50 mm in length. The design of the two robotic arms is optimised so as to dissect surface neoplasms in the digestive tract; therefore, the manipulator arm 3 has the main function of lifting the mucosa and exposing it keeping it under tension by means of a pincer surgical tool 19, while the dissecting arm 4 ends with a mono or bipolar hook-shaped acusector (crochet), that is indicated with reference numeral 20, for removing the neoplastic lesion that is kept lifted and under tension by the pincer surgical tool 19 of the manipulator arm 3.

The actuation of the robotic arms 3 and 4 can in general be obtained with substantially conventional means, or rather by using motors, for example brushless DC motors, that are integrated on the arms themselves or are inside the cap body 1. The movement can be transmitted through mechanical transmission means such as cables, rotation or translation shafts. For the actuation of particular movements, such as for example the opening and closing of pincers or other instruments, it is also possible to use pressurised fluids that are supplied for use through hydraulic ducts.

In a possible embodiment of the invention, schematically illustrated in figure 8, the manipulator arm 3 for lifting and tensioning mucosa is formed by a first portion of manipulator arm 3a, that rigidly protrudes from the crosspiece 13, and by a second portion of manipulator arm 3b, which is connected at its end to the first portion of manipulator arm 3a through an articulation joint 3c, whereas the pincer surgical tool 19 extends from its free end. The manipulator arm 3 has two degrees of freedom. Such degrees of freedom are: pitch (rotation on a transverse axis indicated with Y) that is actuated by means of a motor 21, arranged within the second portion of the manipulator arm 3b, which actuates a geared transmission 3d positioned in the joint 3c; and roll (rotation about the longitudinal axis indicated with X), that is actuated by means of a motor 22, arranged within the first portion of the manipulator arm 3a, the shaft of which is connected to the joint 3c so as to make it carry out controlled angular movements about the axis X. The pincer surgical tool 19 arranged at the end of the arm 3, is actuated by means of a cable (not shown), the tension of which is managed by an electric motor (not shown) which is integrated in the cap body 1. In a variant embodiment not shown, the tension of the cable can be managed by an external electric motor by making, in that case, the cable slide for the entire length of the endoscope 2.

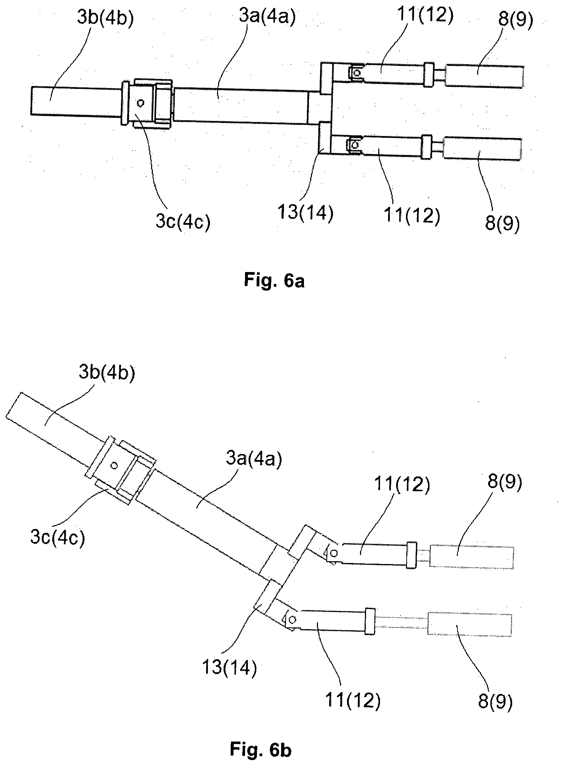

Moreover, as shown in figures 6a and 6b, the manipulator arm 3 can be rotated through the linear actuators 9 about a transverse axis, the path of which is indicated with Z in figure 8, perpendicular to the plane on which the actuators themselves lie. For such a purpose the crosspiece 13, from which the manipulator arm 3 projects, is rotatably connected with its ends 11 to joints and the latter are connected to the shaft of the respective actuators 9, so as to be able to slide axially with respect to the actuators themselves. As shown in figure 6b, different amounts of sliding of the joints 11 corresponds to a rotation of the crosspiece 13 about the axis Z and thus of the manipulator arm 3. This solution makes it possible to develop quite strong forces in relation to the size of the motors that can be used.

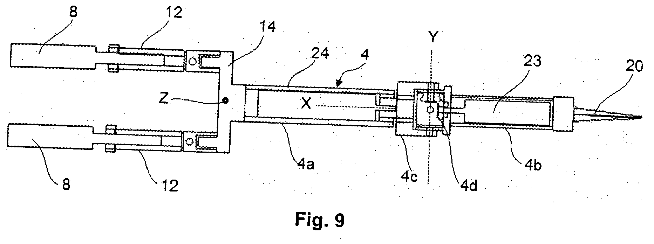

Figure 9 schematically illustrates a possible embodiment of the dissecting arm 4, which is structurally similar to that of the manipulator arm 3. The dissecting arm 4 is formed by a first portion of dissecting arm 4a, rigidly extending from the crosspiece 14, and by a second portion of dissecting arm 4b, which is connected at the end to the first portion of the dissecting arm 4a through an articulation joint 4c, whereas from its free distal end a mono or bipolar hook-shaped tool 20 extends. The dissecting arm 4 has two degrees of freedom: such degrees of freedom are: pitch (rotation on a transverse axis indicated with Y) actuated by means of a motor 23, arranged within the second portion of dissecting arm 4b, which actuates a geared transmission 4d positioned in the joint 4c; and roll (rotation about the longitudinal axis indicated with X), actuated by means of a motor 24, which is arranged within the first portion of dissecting arm 4a, the shaft of which is connected to the joint 4c so as to make it carry out angular movements about the axis X. The hook-shaped tool 20 arranged at the end of the arm 4, is actuated by means of a cable (not shown), the tension of which is managed by an electric motor (not shown) which is integrated inside the cap body 1. In a variant embodiment not shown, the tension of the cable can be managed by an external electric motor in such a case making the cable slide for the entire length of the endoscope 2.

Moreover, as shown in figures 6a and 6b, the dissecting arm 4 can be rotated through the linear actuators 8 about a transverse axis, the path of which is indicated with Z in figure 8, perpendicular to the plane on which the actuators themselves lie. For such a purpose the crosspiece 14, from which the dissecting arm 4 protrudes, is rotatably connected with its ends to the joints 12 and the latter are connected to the shaft of the respective actuators 8, so as to be able to axially slide with respect to the actuators themselves. As shown in figure 6b, different amounts of sliding of the joints 12 corresponds to a rotation of the crosspiece 14 about the axis Z and therefore of the dissecting arm 4. This solution makes it possible to develop quite strong forces in relation to the size of the motors that can be used.

In a second embodiment of the invention, a cable actuation is used for moving the degrees of freedom previously described. In such a case the tension of the cables is controlled by means of motors, such as for example brushless DC motors, which are integrated in the cap body 1.

In a third embodiment of the invention a mixed form of actuation is used consisting of a combination of joints actuated by means of cables, the tension of which is regulated by electric motors present in the cap body 1, and joints that are actuated by electric motors that are coupled by means of suitable gearings with shafts in rotation or translation. These embodiments are not illustrated in detail since they are well known to a man skilled in the art and easily understood from known teachings.

The fixing of the cap body 1 on the endoscope 2 can be made for example by means of adhesive systems possibly in combination with a mechanism that is capable of keeping the adhesion on the surface of the endoscope by exerting on it a light pressure without however damaging its inner structures nor jeopardising its operation. The integration of the cap body 1 on the endoscope 2 requires an increase in the distal diameter up to a maximum of 25 mm.

In the robotic arms 3 and 4 there are angular and linear position sensors that are necessary for controlling them. For their management inside the cap body 1 there are integrated circuits, which are also necessary for controlling the motors integrated in the robotic arms. The electronic circuit boards and the necessary input signals are supplied with power from outside by using cabled transmission systems that slide along the endoscope.

The robotic arms associated with the cap body 1 can be more than two compatibly with the maximum dimensions that the endoscopic device can take up.

The robotic device according to the present invention may be used in an exemplary method for the surgical treatment of pathologies of the digestive tract by means of endoluminal or transluminal surgery operations, in particular of surgical operations for dissection of surface neoplasms, comprising the insertion of the device with the robotic arms in a rest position, where they are retracted inside the cap body, thus being unable to damage the tissues and/or to falter during the insertion towards the operation site; once such site is reached, the present method comprises the actuation of the robotic arms in order for them to slide outside of the cap body and to bring them to a working position, where they are extended and ready to carry out the required manipulation and/or surgical dissection operations. Once the manipulation and/or surgical dissection operations are over, before extracting the device from the patient's body, the robotic arms are actuated again to bring them back in the rest position inside the cap body. Only then the device is extracted, with total safety, maintaining the robotic arms inside the cap body until their extraction is completed.

The main advantage achieved with the present invention is that of making surgical dissections possible by means of a flexible instrument that can be introduced both endoluminally and transluminally. Another advantage consists of the fact that a commercial type of endoscope, at the end of which the robotic device can be simply applied integrally preserving the functionality of the endoscope, can be used as the flexible instrument.

Moreover, with the robotic device according to the invention it is possible to ensure greater accuracy and lower invasiveness in removing neoplastic lesions of the digestive tract since surgical excision can be carried out *en-bloc* with free margins of the superficial lesions, and with a better control of the risk of bleeding and/or perforations. This is obtained thanks to the capability of manipulation of the tissues due to the presence of two active instruments, which allow a surgical gesture that is more precise and natural, allowing actions under visual control that is stable and more accurate. This is given not only by the articulation of the two instruments, but also thanks to the geometry of the device which allows triangulation, i.e. viewing at the centre and manipulation at the sides. In such a manner it is likely to be more difficult for dissection manoeuvres to lead to bleeding and/or perforations, and also in the case in which this happens, the characteristics of greater manipulation of the tissues would allow an easier management of the interoperative complication.

Variants and/or modifications can be carried out to the miniature robotic device designed to be coupled to a flexible endoscope for the surgical dissection of digestive tract surface neoplasms according to the present invention without for this reason departing from the scope of protection of the invention itself as defined in the following claims.

## Claims

1. A miniature robotic device designed to be coupled to a flexible endoscope for the surgical dissection of digestive tract surface neoplasms comprising a cap body (1) removably fixable to a distal end of said endoscope and **characterized in that** it further comprises at least two robotic arms (3, 4), slidably supported within said cap body (1), for performing manipulative and surgical dissection operations and drive means (15) for sliding said arms (3, 4) along said cap body (1) for placing said arms in an operative position, in which they protrude from said cap body (1), and in a rest position, in which they are retracted within said cap body (1).

2. The robotic device according to claim 1, wherein said robotic arms (3, 4) are connected to a base (7) slidable in said cap body (1), said drive means (15) being fixed to said base (7) and arranged between said base (7) and a proximal bottom end of said cap body (1).

3. The robotic device according to claim 2, wherein said robotic arms (3, 4) are connected to said base (7) through means (8, 9) for actuating their movements.

4. The robotic device according to the claim 2 or 3, wherein said drive means comprise a fluidic chamber (15) communicating with an outer, liquid or gas fluid source, and configured so as to expand or contract depending on whether it is filled or emptied of the fluid, whereby its expansion or contraction results in the sliding of said slidable base (7) and the consequent extension out or, respectively, retraction of said arms (3, 4) from/in said cap body (1).

5. The robotic device according to claim 4, wherein said fluidic chamber (15) is substantially bellows-shaped.

6. The robotic device according to any one of the previous claims, wherein said robotic arms are: a manipulator arm (3) having a pincer surgical tool (19) at its end and a dissecting arm (4) having an acusector (20) at its end.

7. The robotic device according to claim 6, wherein said manipulator arm (3) and said dissecting arm (4) each has at least two degrees of freedom.

8. The robotic device according to claim 7, wherein said manipulator arm (3) and said dissecting arm (4) each comprises respective first (3a, 4a) and second (3b, 4b) arm portions articulated to each other, the second arm portions (3b, 4b) carrying the pincer surgical tool (19) and the dissecting tool (20) respectively at their distal ends and being pivotally connected to said first arm portions (3a, 4a) about a first longitudinal axis (X) and a second transverse axis (Y).

9. The robotic device according to claim 8, wherein said manipulator arm (3) and said dissecting arm (4) are further pivotable about a third axis (Z) perpendicular to the plane defined by the rotation axes (X, Y) of said second arm portions (3b, 4b).

10. The robotic device according to claim 9, wherein said manipulator arm (3) and said dissecting arm (4) rigidly extend from respective crosspieces (13, 14) the ends of which are connected to actuator pairs (8, 9) through articulation joints (11, 12), the actuators of each actuator pair being arranged for providing the respective joints (11, 12) with an axial sliding of different extent, thus causing the crosspieces (13, 14) associated thereto to rotate about said third axis (Z).

## Patentansprüche

1. Miniaturisierte robotische Vorrichtung, die gestaltet ist, um an ein flexibles Endoskop für die chirurgische Sezierung von superfiziellen Neoplasmen des Verdauungstraktes, enthaltend einen Kappenkörper (1), der entfernbar an einem distalen Ende des Endoskopes befestigbar ist und **dadurch gekennzeichnet ist, dass** er ferner wenigstens zwei robotische Arme (3, 4), die verschiebbar innerhalb des Kappenkörpers (1) gehalten sind, um manipulative und chirurgische Sezierungsoperationen auszuführen, und Antriebseinrichtungen (15) zum Verschieben der Arme (3, 4) längs des Kappenkörpers (1) enthält, um die Arme in eine operative Position, in der sie von dem Kappenkörper (1) vorstehen, und eine Ruheposition zu versetzen, in der sie nach innerhalb des Kappenkörpers (1) zurückgezogen sind.

2. Robotische Vorrichtung nach Anspruch 1, wobei die robotische Arme (3, 4) mit einer Basis (7) verbunden sind, die in dem Kappenkörper (1) verschiebbar ist, wobei die Antriebseinrichtungen (15) an der Basis (7) befestigt sind und zwischen dxer Basis (7) und einem proximalen Ende des Kappenkörpers (1) angeordnet sind.

3. Robotische Vorrichtung nach Anspruch 2, wobei die robotische Arme (3, 4) mit einer Basis (7) durch Einrichtungen (8, 9) zum Betätigen ihrer Bewegungen verbunden sind.

4. Robotische Vorrichtung nach Anspruch 2 oder 3, wobei die Antriebseinrichtungen eine fluidische Kammer (15) enthalten, die mit einer äußeren Liquid- oder Gasfluid-Quelle in Kommunikation ist und konfiguriert ist, um sich auszudehnen oder zusammenzuziehen in Abhängigkeit davon, ob sie gefüllt ist mit oder geleert ist von dem Fluid, wobei ihre Ausdehnung oder ihr Zusammenziehen in dem Verschieben der verschiebbaren Basis (7) und der konsequenten Verlängerung oder entsprechend Rückziehung der Arme (3, 4) aus dem / in den Kappenkörper (1) resultiert.

5. Robotische Vorrichtung nach Anspruch 4, wobei die fluidische Kammer (15)im Wesentlichen balgförmig ist.

6. Robotische Vorrichtung nach einem der vorhergehenden Ansprüche, wobei die robotischen Arme sind: ein Manipulatorarm (3), der ein chirurgisches Greifer-Werkzeug (19) an seinem Ende hat, und ein Sezierarm (4), der einen Akusektor (20) an seinem Ende hat.

7. Robotische Vorrichtung nach Anspruch 6, wobei der Manipulatorarm (3) und der Sezierarm (4) jeweils wenigstens zwei Freiheitsgrade hat.

8. Robotische Vorrichtung nach Anspruch 7, wobei der Manipulatorarm (3) und der Sezierarm (4) jeweils erste (3a, 4a) und zweite (3b, 4b) Armteile hat, die zueinander gelenkig sind, wobei die zweiten Armteile (3b, 4b) das chirurgisches Greifer-Werkzeug (19) und das Sezier-Werkzeug (20) jeweils an ihren distalen Enden haben und mit den ersten Armteilen (3a, 4a) um eine erste Längsachse (X) und eine zweite Querachse (Y) drehbar verbunden sind.

9. Robotische Vorrichtung nach Anspruch 8, wobei der Manipulatorarm (3) und der Sezierarm (4) ferner um eine dritte Achse (Z) senkrecht zu der Ebene drehbar sind, die durch die Drehachsen (X, Y) der zweiten Armteile (3b, 4b) definiert ist.

10. Robotische Vorrichtung nach Anspruch 9, wobei der Manipulatorarm (3) und der Sezierarm (4) starr von entsprechenden Kreuzstücken (13, 14) ausgehen, deren Enden mit Aktuatorpaaren (8, 9) durch Artikulationsverbindungen (11, 12) verbunden sind, wobei die Aktuatoren jedes Aktuatorpaares angeordnet sind, um die entsprechenden Verbindungen (11, 12) mit einer Axialverschiebung von unterschiedlichem Ausmaß bereitzustellen, wodurch die Kreuzstücke (13, 14), die damit verbunden sind, veranlasst werden, sich um ihre dritte Achse zu drehen.

## Revendications

1. Un dispositif robotique miniature conçu pour être couplé à un endoscope flexible pour la dissection chirurgicale de néoplasmes superficiels du tube digestif comprenant un corps de capuchon (1) pouvant être fixé de manière amovible à une extrémité distale dudit endoscope et **caractérisé en ce qu'**il comprend en outre au moins deux bras robotiques (3, 4), montés de façon coulissante dans ledit corps de capuchon (1), pour effectuer des opérations de dissection chirurgicales et de manipulation et des moyens de ommande (15) pour faire coulisser lesdits bras (3, 4) le long dudit corps de capuchon (1) afin de placer lesdits bras dans en position opérationnelle, dans laquelle ils émergent du dit corps de capuchon (1), et dans une position de repos, dans laquelle ils sont rétractés à l'intérieur du dit corps de capuchon (1).

2. Le dispositif robotique selon la revendication 1, dans lequel lesdits bras robotiques (3, 4) sont reliés à une embase (7) apte à coulisser dans ledit corps de capuchon (1), lesdits moyens de commande (15) étant fixés à ladite embase (7) et agencés entre ladite embase (7) et une extrémité de fond proximale du dit corps de capuchon (1).

3. Le dispositif robotique selon la revendication 2, dans lequel lesdits bras robotiques (3, 4) sont reliés à ladite embase (7) par l'intermédiaire de moyens (8, 9) pour actionner leurs mouvements.

4. Le dispositif robotique selon l'une des revendications 2 et 3, dans lequel lesdits moyens de commande comprennent une chambre fluidique (15) communiquant avec une source extérieure de fluide liquide ou gazeux, et configurée pour s'étendre ou se contracter selon qu'elle est remplie de fluide ou vidée, de façon que son expansion ou sa contraction se traduise par le glissement de ladite embase coulissante (7) et en conséquence l'extension vers l'extérieur ou, respectivement, la rétraction desdits bras (3, 4) à partir du dit corps de capuchon / dans ledit corps de capuchon (1).

5. Le dispositif robotique selon la revendication 4, dans lequel ladite chambre fluidique (15) est en forme sensiblement de soufflet.

6. Le dispositif robotique selon l'une quelconque des revendications précédentes, dans lequel lesdits bras robotiques sont : un bras de manipulation (3) ayant un outil chirurgical à pince (19) à son extrémité et un bras de dissection (4) ayant un bistouri électrique (20) à son extrémité.

7. Le dispositif robotique selon la revendication 6, dans lequel ledit bras de manipulation (3) et ledit bras de dissection (4) ont chacun au moins deux degrés de liberté.

8. Le dispositif robotique selon la revendication 7, dans lequel ledit bras de manipulation (3) et ledit bras de dissection (4) comprennent chacun respectivement des première (3a, 4a) et seconde (3b, 4b) portions de bras articulées l'une par rapport à l'autre, les secondes portions de bras (3b, 4b) portant l'outil chirurgical à pince (19) et l'outil de dissection (20) respectivement à leurs extrémités distales et étant reliées aux dites premières portions de bras (3a, 4a) de manière pivotante autour d'un premier axe longitudinal (X) et d'un second axe transversal (Y).

9. Le dispositif robotique selon la revendication 8, dans lequel ledit bras de manipulation (3) et ledit bras de dissection (4) peuvent en outre pivoter autour d'un troisième axe (Z) perpendiculaire au plan défini par les axes de rotation (X, Y) des dites secondes portions de bras (3b, 4b).

10. Le dispositif robotique selon la revendication 9, dans lequel ledit bras de manipulation (3) et ledit bras de dissection (4) s'étendent de manière rigide à partir de traverses respectives (13, 14) dont les extrémités sont connectées à des paires d'actionneurs (8, 9) par l'intermédiaire de joints d'articulation (11, 12), les actionneurs de chaque paire d'actionneurs étant agencés de façon à conférer aux joints respectifs (11, 12) un coulissement axial d'extension différente, entraînant ainsi la rotation des traverses (13, 14) associées à ceux-ci autour du dit troisième axe (Z).
